# FASCICULE DE BREVET EUROPEEN

(11) **EP 0 375 751 B1**
(45) Date de publication et mention de la délivrance du brevet: **04.08.1993**
(21) Numéro de dépôt: 89904570.2
(22) Date de dépôt: 10.04.1989
(51) Int. Cl.: C12Q 1/04, C12Q 1/64, C12M 1/34

(54) **PROCEDE ET DISPOSITIF POUR LA DETECTION DE BACTERIES SULFATO-REDUCTRICES**
VERFAHREN UND VORRICHTUNG ZUM NACHWEIS DER SULFATREDUZIERENDEN BAKTERIEN
METHOD AND DEVICE FOR DETECTING SULPHATE-REDUCING BACTERIA

(30) Priorité: 11.04.1988 FR 8804724
(43) Date de publication de la demande: 04.07.1990
(73) Titulaire: ELF AQUITAINE PRODUCTION, 92400 Courbevoie (FR)
(72) Inventeur: SEUREAU, Jacques, F-31420 Aurignac (FR); MONDEIL, Lucien, F-64160 Serres-Morlaas (FR); AUSSEUR, Joelle, F-78000 Versailles (FR); MAGOT, Michel Lotissement des Chanterelles, F-31650 Saint-Orens-Gameville (FR); PROTIN, Jean, F-64110 Mazères-Lezon (FR); SOURBE, Jean, F-64150 Noguères (FR)
(74) Mandataire: Debay, Yves
(86) Numéro de dépôt international: FR8900164
(87) Numéro de publication internationale: WO8909832

(56) Documents cités:
- WO-A-83/01071
- GB-A- 1 228 359

## Description

La présente invention concerne un procédé de détection de bactéries sulfato-réductrices, un dispositif de détection automatique de ces bactéries et l'application du procédé.

Lorsque l'on soutire du pétrole dans un gisement on obtient un meilleur taux de récupération des reserves en place en injectant dans la roche, par un puits spécialisé une quantité d'eau égale à la quantité de pétrole prélevée afin de maintenir la pression dans le gisement.

Cette eau est filtrée soigneusement, de façon à contenir des particules de taille inférieure à 3 µm pour ne pas boucher les pores de la roche autour du puits d'injection. Cette eau est également très sérieusement désaérée pour ne pas risquer d'oxydation dans la conduite d'injection. Ce faisant elle devient ainsi un milieu anaérobié permettant le développement des bactéries sulfato-réductrices présentes dans toute eau douce dans le cas d'un champ à terre ou salée dans le cas d'un champ en mer. La présence des bactéries est surveillée par prélèvements journaliers, cultures et examens, et en fonction de ces résultats, on empêche le développement intempestif des bactéries par injection de produit anti-bactérien spécifique ou par iradiation aux ultra-violets.

Il est connu par la demande de brevet européen 0 242 082 un procédé de détection et de dénombrage des bactéries sulfato-réductrices consistant à immobiliser les cellules bactériennes sur un support par l'application d'un agent d'immobilisation choisi dans une suspension d'hydroxyde de titane. Ce procédé nécessite des opérations nombreuses telles que l'extraction par centrifugation de l'échantillon à tester, la dilution de celui-ci dans une solution de chlorure de sodium, la fixation par hydroxyde de titane, un triple lavage dans de la caseïne, une incubation, l'addition d'un enzyme et une nouvelle incubation pour permettre à une couleur de se développer et une détection possible par absorption d'une raie de 0,45 µm. Un tel procédé présente l'inconvénient de nécessiter de multiples opérations qui conduisent difficilement à un processus automatisable et à la constitution d'un dispositif automatique permettant de réduire la charge de travail du personnel en plate-forme.

Il est également connu par le brevet britannique 1 228 359, une méthode de tests de la concentration de micro-organismes anaérobie dans une émulsion d'huile de machine par l'ensemencement de cette émulsion à l'aide d'un milieu nutritif comprenant une source soufrée et une source de fer et l'incubation du milieu ensemencé pour détecter par l'apparition d'un précipité noir dû au sulfate de fer des bactéries.

Un premier but de l'invention est donc d'améliorer le procédé de détection par sulfate de fer ci-dessus en vue de son application à l'exploitation des gisements pétroliers.

Ce premier but est atteint par le procédé de l'invention qui est caractérisé en ce que le procédé de détection par sulfate de fer des bactéries sulfato-réductrices selon l'art antérieur comporte en outre un milieu nutritif contenant deux sources de carbone, un indicateur d'oxydo-réduction, un réducteur non soufré, une source d'azote, de l'extrait de levure, l'ensemencement du milieu réactif étant effectué par l'eau d'un gisement, la détection des bactéries sulfato-réductrices étant effectuée par spectro-photométrie.

Selon une autre caractéristique, l'atmosphère anaérobie est constituée d'un gaz neutre plus lourd que l'air, tel que de l'argon.

Selon une autre caractéristique la source de carbone est un lactate et un acétate de sodium.

Selon une autre caractéristique, l'oxydo-réducteur est de la résazurine.

Selon une autre caractéristique, le réducteur non soufré est du citrate de titane.

Selon une autre caractéristique, la source d'azote est du chlorure d'ammoniaque.

Un autre but de l'invention est de proposer un procédé permettant de vérifier l'anaérobiose.

Ce but est atteint par le fait que la vérification de l'anaérobiose est effectuée par analyse spectro-photométrique de récipients témoins contenant le même milieu nutritif, additionné d'eau distillée stérile à l'aide d'un spectro-photomètre centré sur la raie d'absorption de l'indicateur d'oxydo-réduction.

Un autre but est de proposer un dispositif de détection de bactéries, nécessitant un minimum de manipulations et pouvant fonctionner en automatique.

Ce but est atteint par le fait que le dispositif de détection de bactéries sulfato-réductrices comporte des moyens de génération et maintien d'une atmosphère anaérobie :
- des moyens de détection de la qualité anaérobie de l'atmosphère;
- un ensemble d'éléments porteurs de tubes bouchés contenant un liquide nutritif, des réactifs de l'action métabolique des bactéries sulfato-réductrices et le contrôle de l'anaérobiose de l'eau;
- un deuxième ensemble porteur de tubes ensemencés et en culture à une température constante;
- un dispositif d'ensemencement par un dispositif automatique de prise d'une dose prédéterminée d'eau d'injection;
- un dispositif de détection permettant la détection de l'opacification de la solution contenue dans les tubes;
- un système de manipulation automatique des tubes et de présentation devant le dispositif d'ensemencement et le dispositif de détection;
- un dispositif de bouchage et de débouchage des tubes.

Selon une autre caractéristique, les moyens de détection de la qualité anaérobie sont constitués par un troisième ensemble de tubes contenant le même liquide nutritif additionné d'eau distillée stérile et de résazurine.

Selon une autre caractéristique, le système de manipulation comporte un robot manipulateur et son électronique de commande et un dispositif permettant au robot de se recaler périodiquement pour entrer ses dérives de position dans le calcul des coordonnées des mouvements à effectuer.

Un autre but est de proposer un dispositif qui puisse continuer à fonctionner entre deux périodes de maintenance.

Ce but est atteint par le fait que le dispositif de détection est constitué d'un double spectro-photomètre, centré chacun sur la raie de 0,56 µm de la résazurine et dont le second est mis en fonction sur détection de la défaillance du premier.

Selon une autre caractéristique le dispositif d'ensemencement est constitué d'un dispositif de dosage automatique avec détection du bon fonctionnement comprenant une vanne (V1) de distribution de l'eau d'alimentation à un pot comportant à son extrémité supérieure un tuyau d'évacuation du trop-plein et à son extrémité inférieure un tuyau de distribution par une deuxième vanne (V2), un élément collecteur débouchant dans une canalisation d'évacuation équipée d'un détecteur de débit.

Enfin un dernier but de l'invention est l'application du procédé.

Cette application permet la détermination de la présence de bactéries sulfato-réductrices dans une eau anaérobie prélevée dans un gisement et le déclenchement à partir de cette détection des actions anti-bactériennes.

D'autres caractéristiques et avantages de la présente invention apparaîtront plus clairement à la lecture de la description ci-après faite en liaison avec les figures dans lesquelles :
- la figure 1 représente une vue schématique d'ensemble du dispositif permettant la mise en oeuvre du procédé selon l'invention;
- la figure 2 représente une vue schématique du dispositif de dosage mis en oeuvre à la figure 1.

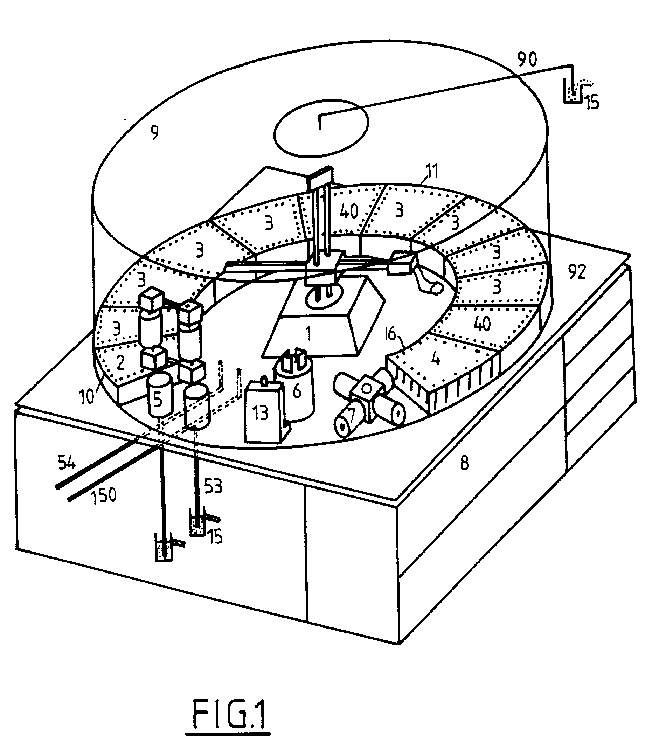

Le dispositif permettant la mise en oeuvre du procédé selon l'invention et représenté à la figure 1 comporte, dans une cloche étanche (9) supportée par un plateau (92) un robot manipulateur (1) équipé d'un système (13) permettant au robot de se recaler dans l'espace périodiquement de façon à pouvoir entrer ses dérives de position dans le calcul des coordonnées des divers mouvements à effectuer. Un premier ensemble (3) d'éléments porteurs de tubes (11) contient un liquide nutritif et le réactif de l'action métabolique des bactéries sulfato-réductrices. Un deuxième ensemble (4) comporte des tubes ensemencés qui sont mis en culture, par exemple, à une température de 30 degrés Celsius pendant un temps d'incubation pouvant aller, par exemple, jusqu'à 22 jours. Cet ensemble (4) a son chauffage assuré par une sole chauffante dans laquelle sont encastrés des cylindres permettant l'introduction des tubes. La conduction de la chaleur par les cylindres alliée à une régulation en tout ou rien de la température de la sole à l'aide d'une sonde thermique permet le maintien de la température à plus ou moins un quart de degré Celsius près. L'appareil comporte également un dispositif spectro-photométrique (7) constitué d'un double photomètre dont un élément est utilisé tandis que l'autre est conservé en réserve, les deux éléments étant centrés sur la raie de 0,56 µm permettant l'examen du virage de la solution de résazurine. Une station (6) de vissage et de dévissage des bouchons des tubes (11) est également présente.

Le dispositif comporte également dans la cloche (9) étanche dans ses points bas et possèdant une sortie haute (90) permettant le maintien d'une atmosphère anaérobie, des syphons (15) de protection contre des entrées intempestives de l'air ambiant et permettant d'alimenter la cloche en gaz neutre plus lourd que l'air tel que de l'argon. Cette alimentation est assurée par la canalisation (150). Dans la cloche se trouve également un système automatique (5) de dosage d'une quantité déterminée d'eau d'injection prélevée dans le gisement en vue de la détection des bactéries. Enfin le dispositif comporte un troisième ensemble d'éléments porteurs (2) de tubes (10) dits "tubes témoins" contenant le même liquide nutritif, de la résazurine et de l'eau distillée stérile. Ces tubes témoins permettent de vérifier que l'atmosphère à l'intérieur de la cloche est anaérobie. A l'extérieur de la cloche se trouve une électronique (8) de commande des déplacements du bras manipulateur, de la sélection d'un des deux photomètres, de régulation de la puissance de chauffe de l'ensemble (4) d'incubation, d'amplification des signaux du spectromètre et de commande des vannes du dispositif de dosage, selon la séquence explicitée plus loin.

Les tubes (11) contiennent un liquide nutritif dont la préparation est effectuée selon le processus suivant.

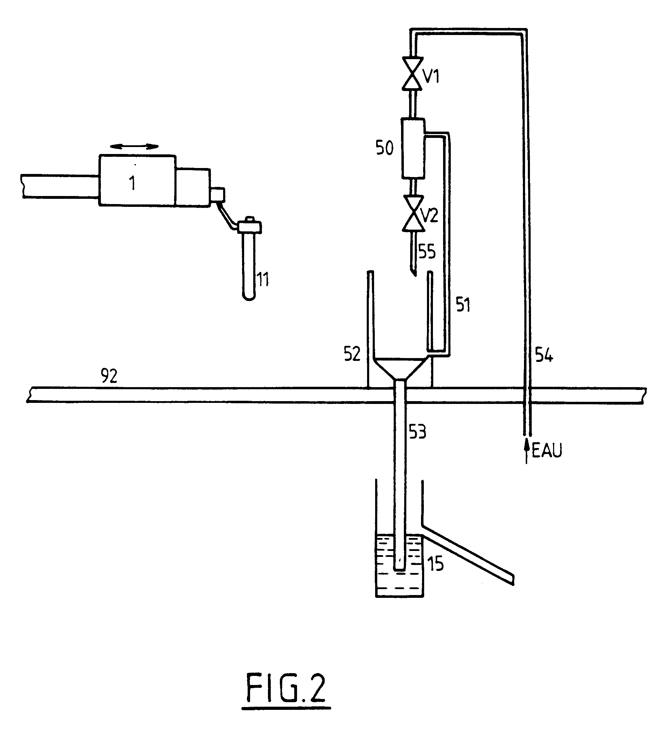

On dissout dans un litre d'eau distillée 10 g d'extrait de levure, 10 g de chlorure d'ammoniaque NH₄Cl, 60 ml de lactate de sodium, 20 ml d'acétate de sodium, 10 ml d'une solution de résazurine a O,1% dans l'eau. La solution est répartie dans des tubes de type plasma qui sont placés dans une enceinte anaérobie pendant quatre heures. Les tubes sont ensuite bouchés puis autoclavés 20 minutes à 120 degrés Celsius. Après avoir rentré à nouveau les tubes dans l'enceinte anaérobie, on ajoute 4% d'une solution de citrate de titane. Cette solution est obtenue à partir de 10 ml de chlorure de titane à 15% dans 100 ml de citrate de sodium O,2N ayant un pH de 6, stérilisé par filtration sur une membrane de O,2 microns. Le pH du milieu ainsi obtenu est ajusté à la valeur de 7,4 par de la soude 2N stérile. On ajoute ensuite 5% de sulfate de fer FeSO₄ en solution à 2,5% dans l'eau stérilisée par filtration sur une membrane de O,2 microns. Les tubes (11) sont préalablement déaérés 24 heures dans la chambre anaérobie, bouchés et stérilisés par autoclavage de 20 minutes à 120 degrés Celsius. Puis dans la chambre anaérobie on répartit 1 ml du milieu nutritif concentré stérile obtenu précédemment dans chacun des tubes stériles (11) qui sont ensuite hermétiquement bouchés pour être stockés à l'air. Les tubes témoins (10) d'anaérobiose sont préparés de la même façon que les tubes précédents mais contiennent en plus du millilitre de milieu nutritif concentré, 10 ml d'eau distillée stérile réduite par 2% de solution de citrate de titane. Le dispositif de dosage permettant l'ensemencement représenté à la figure 2 comporte la canalisation (54) d'arrivée d'eau en provenance du gisement et une vanne (V1) permettant à cette eau d'alimenter un pot (50) de contenance en rapport avec la quantité à délivrer. Ce pot (50) comporte dans sa partie supérieure une canalisation (51) d'évacuation de la quantité d'eau supérieure à la quantité à doser et à sa partie inférieure une canalisation débouchant par une vanne (V2) sur un dispositif de récupération (52). Le dispositif de récupération des eaux (52) débouche par une canalisation d'évacuation (53) sur un syphon (15). Le dispositif de dosage décrit permet ainsi d'effectuer un prélèvement automatique journalier de l'eau en provenance du gisement tout en évitant d'arrêter l'écoulement de l'eau de façon prolongée dans le circuit de prélèvement pour empêcher l'établissement de cultures de bactéries sulfato-réductrices dans le circuit d'eau. Le volume constant d'eau est obtenu à l'aide des deux électrovannes (V1, V2) par remplissage du réservoir (50) au niveau constant représenté par le circuit d'évacuation (51). Le cycle de fonctionnement commandé par l'électronique est le suivant. Dans un premier temps les vannes (V1 et V2) sont ouvertes et l'eau s'écoule directement dans le syphon (52) de vidange. Puis la vanne (V1) reste ouverte, la vanne (V2) est fermée et le réservoir (50) se remplit jusqu'à la hauteur de la canalisation (51). Au bout d'un certain temps (V1) est fermée, (V2) restant fermée, le trop-plein d'eau s'écoule par la canalisation (51). Enfin lorsqu'un tube (11) est amené sous la canalisation (55) de la vanne (V2), la vanne (V1) étant fermée et la vanne (V2) étant ouverte, le volume déterminé s'écoule dans le tube et ensemence le milieu nutritif.

Le double spectro-photomètre (7) est étalonné chaque jour en mesurant le flux lumineux reçu par une photodiode en l'absence de tube. Cette mesure permet d'ajuster le niveau de l'alimentation de la lampe pour obtenir toujours la même valeur, ceci de façon à mesurer valablement la transmission d une culture au cours du temps. Dans le cas où l'étalonnage indique que l'un des spectro-photomètre est en panne, le dispositif électronique (8) lance le fonctionnement de l'autre et commute les organes nécessaires pour celà. Le spectro-photomètre est utilisé pour vérifier que les circuits de prélèvement d'eau ont bien fonctionné en présentant le tube (11) contenant le liquide nutritif au spectro-photomètre avant l'ensemencement, ce qui donne une valeur de transmission (T0), puis après ensemencement, ce qui doit donner une valeur de transmission (T1) différente de (T0). Dans le cas contraire, ceci indique un défaut de fonctionnement du circuit de prélèvement. Le spectro-photomètre permet également de vérifier que l'atmosphère est toujours anaérobie en mesurant la transmission d'un tube témoin (10) testé périodiquement par mise du réactif en contact avec l'atmosphère à l'intérieur de la cloche puis mesure au photomètre pour détecter le virage au rouge de la résazurine dans le cas où l'atmosphère est anaérobie.

Le spectro-photomètre permet également de détecter la présence de bactéries dans les tubes (11) ensemencés et soumis à incubation par la perte presque totale de la transmission dans le tube quand le sulfure de fer a précipité.

Le fonctionnement du dispositif est le suivant.

Le bras du robot (1) prend le tube (11) par le bouchon dans la zone de stockage (3), le pose sur le poste de vissage-dévissage (6) qui emprisonne le tube fermement puis tourne le tube pour dévisser le bouchon tandis que le bras du robot (1) maintient ce bouchon. Le bras pose le bouchon puis saisit le tube (11) qui est alors libéré par la station de vissage (6). Le bras présente le tube au poste d'ensemencement (5) qui délivre la dose prédéterminée d'eau puis ramène le tube sur la station de vissage-dévissage (6). Cette station (6) se referme sur le tube et le bras prend le bouchon pour l'amener en vis-à-vis du tube ouvert. La station (6) entrainée en rotation permet le rebouchage du tube (11). Après libération du tube par la station et agitation du tube ensemencé par le bras, ce dernier pose le tube ensemencé (16) dans la zone de culture (4) où la température est maintenue constante. Chaque jour le bras prend les tubes (16) en incubation dans la zone de culture pour examen au spectro-photomètre et recommence la séquence pour ajouter un nouveau tube ensemencé.

Sur détection d'un précipité de sulfure de fer noir ou après un nombre de jours prédéterminé de culture le tube ensemencé est mis dans une seconde zone de stockage (40).

Pour effectuer l'ensemencement, l'électronique du robot commande au poste de prélèvement de fermer la vanne (V2) pendant trente secondes puis de fermer (V1) et d'attendre. Le bras amène le tube sous l'extrémité (55) et (V2) est ouvert. Dès l'ouverture, le tube se remplit puis est remonté légèrement pour être amené en contact avec l'extrémité du tube (55) taillée en biseau de façon à ramasser la dernière goutte. Le tube ensemencé (16) est 5 alors retiré du poste de prélèvement. Dans le cas où l'on souhaite avoir à assurer la maintenance de l'installation tous les 22 jours, le poste d'incubation comporte 21 positions de rangement des tubes ensemencés (16). Chaque jour le bras après mesure au spectro-photomètre, décale 10 tous les tubes d'un cran dans le poste d'incubation (4). Ainsi le tube du premier jour passe à la deuxième position est ainsi de suite. Après la 21ème position, quelque soit le résultat de la mesure, le tube est placé sur la portion de stockage (40). De même si un tube a mis en évidence la 15 présence de bactéries avant le 21ème jour il est également sorti du portoir de culture (4). L'emplacement vide résultant demeurera mais sera déplacé chaque jour comme les tubes. De cette façon, chaque jour la première position, de l'élément porteur permettant l'incubation (4), est utilisée 20 pour entamer une nouvelle culture.

Le dispositif ainsi décrit permet le fonctionnement automatique du dispositif pendant une durée correspondant à la capacité maximale du nombre de tubes (11) à ensemencer placés dans une enceinte. De cette façon, 25 la difficulté de décontaminer et stériliser les tubes a été transférée à la préparation en usine alors que les tubes fermés sont mis en place dans l'enceinte à chaque période de maintenance, idéalement tous les ans, par un personnel qui n'a pas besoin de formation spécifique. La mise sous 30 argon de l'enceinte permet de réaliser facilement une atmosphère anaérobie, sans avoir besoin de faire appel à un processus de mise sous vide qui reste complexe. La sensibilité de la détection a par ailleurs été améliorée grâce à l'utilisation de 1 ml par tube de milieu nutritif 35 concentré dix fois et dilué dans 10ml d'eau lors de l'ensemencement. De plus pour tenir compte de la diversité métabolique des bactéries sulfato-réductrices, on a ajouté deux sources de carbone au milieu nutritif. Enfin l'indicateur coloré d'oxydo-réduction constitué par la résazurine permet de vérifier l'anaérobiose de la cloche.

## Revendications

1. Procédé de détection de bactéries sulfato-réductrices par utilisation de la méthode de culture des bactéries sulfato-réductrices comprenant l'ensemencement par injection en anaérobiose d'une quantité constante de prélèvements à tester dans un récipient contenant un milieu nutritif préréduit concentré et contenant une source de fer, l'incubation du milieu ensemencé à une température constante, caractérisé en ce que le milieu nutritif est concentré 10 fois et comprend en outre deux sources de carbone, un indicateur d'oxydo-réduction, un réducteur non soufré, une source d'azote, de l'extrait de levure, l'ensemencement est effectué par l'eau d'un gisement, la détection des bactéries sulfato-réductrice est effectuée par spectro-photométrie.

2. Procédé selon la revendication 1, caractérisé en ce que l'atmosphère anaérobie est constituée d'un gaz neutre plus lourd que l'air.

3. Procédé selon la revendication 2, caractérisé en ce que le gaz est de l'argon.

4. Procédé selon la revendication 1, caractérisé en ce que la double source de carbone est un lactate et un acétate de sodium.

5. Procédé selon la revendication 1, caractérisé en ce que l'oxydo-réducteur est de la résazurine.

6. Procédé selon la revendication 1, caractérisé en ce que le réducteur non soufré est du citrate de titane.

7. Procédé selon la revendication 1, caractérisé en ce que la source d'azote est du chlorure d'ammoniaque.

8. Procédé selon la revendication 1 ou 5, caractérisé en ce qu'il comprend la vérification de l'anaérobiose par analyse spectro-photomètrique de récipients témoins (10) contenant le même milieu nutritif additionné d'eau distillée stérile à l'aide d'un spectro-photomètre centré sur la raie d'absorption de l'indicateur d'oxydo-réduction.

9. Procédé selon la revendication 8, caractérisé en ce que la détection des bactéries sulfato-réductrices est effectuée sur l'apparition d'un précipité noir de sulfure de fer absorbant le flux lumineux du spectro-photomètre.

10. Dispositif de détection de bactéries sulfato-réductrices, caractérisé en ce qu'il comporte :
- des moyens de génération et de maintien d'une atmosphère anaérobie;
- des moyens de détection de la qualité anaérobie de l'atmosphère;
- un ensemble d'éléments (3) porteurs de tubes (11) contenant un liquide nutritif, le réactif de l'action métabolique des bactéries sulfato-réductrices et le réactif de contrôle de l'anaérobiose de l'eau;
- un deuxième ensemble (4) porteur de tubes ensemencés (16) en culture à une température constante;
- un dispositif d'ensemencement (5) par un dispositif automatique de prise d'une dose prédéterminée d'eau d'injection ;
- un dispositif spectro-photométrique (7) permettant la détection de l'opacification de la solution contenue dans les tubes ;
- un système de manipulation automatique (1) des tubes et de présentation devant le dispositif d'ensemencement (5) et le dispositif de détection (7);
- un dispositif de bouchage et de débouchage (6) des tubes.

11. Dispositif selon la revendication 10, caractérisé en ce que les moyens de détection de la qualité anaérobie sont constitués par un troisième ensemble de tubes (10) contenant le même liquide nutritif additionné d'eau distillée stérile et de résazurine.

12. Dispositif selon la revendication 10 ou 11, caractérisé en ce que les systèmes de manipulation comportent un robot manipulateur (1) et son électronique (8) de commande, et un dispositif (13) permettant au robot de se recaler périodiquement pour entrer ses dérives de position dans le calcul des coordonnées des mouvements à effectuer.

13. Dispositif selon l'une des revendications 10 à 12, caractérisé en ce que le dispositif de détection est constitué d'un double photomètre centré chacun sur la raie 0,56 µm de la résazurine et dont le second est mis en fonction par le circuit électronique (8) sur détection de la défaillance du premier.

14. Dispositif selon une des revendications 10 à 13, caractérisé en ce que le dispositif d'ensemencement est constitué d'un dispositif de dosage automatique (5) avec détection de bon fonctionnement comprenant une vanne (V1) de distribution de l'eau d'alimentation à un pot (50) comportant à son extrémité supérieure un tuyau (51) d'évacuation du trop plein et à son extrémité inférieure un tuyau (55) de distribution par une deuxième vanne (V2), un élément (52) collecteur débouchant dans une canalisation (53) d'évacuation.

15. Application du procédé et/ou du dispositif selon une des revendications précédentes, caractérisé en ce qu'il permet la détermination de la présence de bactéries sulfato-réductrices dans une eau anaérobie prélevée dans un gisement pétrolier et le déclenchement à partir de cette détection des actions anti-bactériennes.

## Claims

1. Method for detecting sulfato-reducing bacteria by using the method for cultivating sulfato-reducing bacteria including the anaerobic injection sowing of a constant quantity of samplings to be tested in a vessel containing a concentrated prereduced nutritive medium and an iron source, the incubation of the sowed medium being effected at a constant temperature, wherein the nutritive medium is concentrated 10 times and further includes two carbon sources, an oxdation-reduction indicator, a non-sulfurous reducing agent, a nitrogen source, yeast extract, sowing being effected through the water of a deposit and detection of the sulfato-reducing bacteria being effected by spectrophotometry.

2. Method according to claim 1, wherein the anaerobic atmosphere is formed of a neutral gas heavier than air.

3. Method according to claim 2, wherein the gas is argon.

4. Method according to claim 1, wherein the double carbon source is lactate and sodium acetate.

5. Method according to claim 1, wherein the oxidation-reductor is resazurin.

6. Method according to claim 1, wherein the non-sulfured reducing agent is titanium citrate.

7. Method according to claim 1, wherein the nitrogen source is ammonium chloride.

8. Method according to claim 1 or 5, wherein it includes checking of anaerobiosis by the spectrophotometric analysis of test specimen vessels (10) containing the same nutritive medium with sterile distilled water added with the aid of a spectrophotometer centered on the absorption line of the oxidation-reduction indicator.

9. Method according to claim 8, wherein the detection of the sulfato-reducing bacteria is effected upon the appearance of a black precipitate of iron sulfur absorbing the luminous flux of the spectrophotometer.

10. Device for detecting sulfato-reducing bacteria and wherein it comprises :
- means for generating and maintaining an anaerobic atmosphere ;
- means for detecting the anaerobic quality of the atmosphere ;
- a set of elements (3) carrying tubes (11) containing a nutritive liquid, the reagent of the metabolic action of the sulfato-reducing bacteria and the reagent for controlling the anaerobiosis of the water ;
- a second tube carrier unit (4) culture-sowed at a constant temperature ;
- a device (5) for sowing by means of an automatic device for picking up a predetermined dose of injection water ;
- a spectrophotometric device (7) able to detect the opacification of the solution contained in the tubes ;
- a system (1) for the automatic handling of the tubes and placed in front of the sowing device (5) and the detection device (7) ;
- a device (6) for blocking and unblocking tubes.

11. Device according to claim 10, wherein the means for detecting the anaerobic quantity are constituted by a third set of tubes (10) containing the same nutritive liquid added with sterile distilled water and resazurin.

12. Device according to claim 10 or 11, wherein the handling systems comprise a handling robot (1) and its control electronics (8), and a device (13) enabling the robot to periodically be reset so as to enter its position derivatives in the calculation of the coordinates of the movements to be made.

13. Device according to any one of claims 10 to 12, wherein the detection device is constituted by a double photometer with each portion being centered on the 0.56 µm line of the resazurin, the second photometer being instigated by the electronic circuit (8) on detection of a malfunctioning of the first photometer.

14. Device according to any one claims 10 to 13, wherein the sowing device is formed of an automatic dosing device able to detect proper functioning and including a distribution valve (V1) for water-feeding a crucible (50) comprising at its upper extremity an overflow evacuation pipe (51) and at its lower extremity a pipe (55) for distribution via a second valve (V2), and a collecting element (52) opening into an evacuation pipe (53).

15. Application of the method and/or the device according to any one of the preceding claims, wherein it makes it possible to determine the presence of sulfato-reducing bacteria in anaerobic water sampled from a petroleum deposit and to release it on the basis of this detection of antibacteria actions.

## Patentansprüche

1. Verfahren zur Erkennung von Sulfato-Reduktionsbakterien durch Anwendung der Methode der Sulfato-Reduktionsbakterienkultur unter Einimpfen einer konstanten Menge von zu prüfenden Proben in anaerober Atmosphäre in einen Behälter mit einem vorreduzierten, konzentrierten Nährmedium, einer Eisenquelle und anschließender Inkubation des geimpften Nährmediums bei konstanter Temperatur, dadurch gekennzeichnet, daß das Nährmedium zehnfach konzentriert ist und weiterhin zwei Kohlenstoffquellen, einen Oxidations-Reduktionsindikator, ein schwefelfreies Reduktionsmittel, eine Stickstoffquelle und Hefeextrakt enthält, wobei die Einimpfung durch das Wasser eines Erdölvorkommens und die Erkennung der Sulfato-Reduktionsbakterien durch Spektralphotometrie erfolgt.

2. Verfahren nach Patentanspruch 1, dadurch gekennzeichnet, daß die anaerobe Atmosphäre aus einem neutralen Gas besteht, das schwerer als Luft ist.

3. Verfahren nach Patentanspruch 1, dadurch gekennzeichnet, daß dieses Gas aus Argon besteht.

4. Verfahren nach Patentanspruch 1, dadurch gekennzeichnet, daß die doppelte Kohlenstoffquelle aus Laktat und Natriumacetat besteht.

5. Verfahren nach Patentanspruch 1, dadurch gekennzeichnet, daß das Oxidations-Reduktionsmittel aus Resazurin besteht.

6. Verfahren nach Patentanspruch 1, dadurch gekennzeichnet, daß das schwefelfreie Reduktionsmittel aus Titanzitrat besteht.

7. Verfahren nach Patentanspruch 1, dadurch gekennzeichnet, daß die Stickstoffquelle aus Ammoniakchlorid besteht.

8. Verfahren nach Patentanspruch 1 oder 5, dadurch gekennzeichnet, daß er eine Überprüfung der Anaerobiose durch Spektralphotometrie von Vergleichsprobenbehältern (10) beinhaltet, die denselben, jedoch mit sterilem destilliertem Wasser angereicherten Nährmedium enthalten, wobei das Spektralphotometer auf die Absorptionslinie des Oxidations-Reduktionsindikators ausgerichtet wird.

9. Verfahren nach Patentanspruch 8, dadurch gekennzeichnet, daß die Erkennung der Sulfato-Reduktionsbakterien bei Auftreten eines schwarzen Ausfalls von Eisensulfid erfolgt, durch das der Lichtstrahl des Spektralphotometers absorbiert wird.

10. Vorrichtung zur Erkennung von Sulfato-Reduktionsbakterien, dadurch gekennzeichnet, daß sie aus folgenden Teilen besteht:
- Mittel zum Erzeugen und Beibehalten einer anaeroben Atmosphäre,
- Mittel zur Erkennung des Anaerobiewertes der Atmosphäre,
- einer Baugruppe (3) mit Elementen zum Festhalten von Reagenzgläsern (11), die eine Nährflüssigkeit, das Reagens für die Stoffwechselvorgänge der Sulfato-Reduktionsbakterien und das Reagens zur Prüfung der Anaerobiose des Wassers enthalten,
- einer weiteren Baugruppe (4) zum Festhalten von Reagenzgläsern (11) mit eingeimpften und bei konstanter Temperatur gehaltenen Kulturen,
- einer Vorrichtung zum Einimpfen (5) durch automatisches Entnehmen einer vorgegebenen Menge einzuimpfenden Wassers,
- einer spektralphotometrischen Vorrichtung (7) zur Erkennung der Trübung der Flüssigkeit in den Reagenzgläsern,
- einem Handhabungssystem zum automatischen Befördern (1) der Reagenzgläser zu der Einimpfvorrichtung (5) und der Erkennungsvorrichtung (7),
- einer Vorrichtung zum Verschließen und Öffnen (6) der Reagenzgläser.

11. Vorrichtung nach Patentanspruch 10, dadurch gekennzeichnet, daß die Vorrichtung zur Erkennung des Anaerobiewertes aus einer dritten Baugruppe (10) zum Festhalten von Reagenzgläsern besteht, die dieselbe, jedoch mit sterilem destilliertem Wasser und Resazurin angereicherte Nährlösung enthalten.

12. Vorrichtung nach Patentanspruch 10 oder 11, dadurch gekennzeichnet, daß das Handhabungssystem aus einem Handhabungsroboter (1) mit der zugehörigen Steuerelektronik (8) sowie einer Vorrichtung (13) besteht, anhand der der Roboter sich regelmäßig neu einstellen kann, um seine Positionsabweichungen in die Berechnung für die auszuführenden Bewegungen einzugeben.

13. Vorrichtung nach einem der Patentansprüche 10 bis 12, dadurch gekennzeichnet, daß die Erkennungsvorrichtung aus einem zweifachen Photometer besteht, wobei jedes davon auf die Spektrallinie des 0,56 µm breiten Resazurins ausgericht ist und das zweite Photometer durch die Elektronikschaltung (8) bei Erkennung eines Ausfalls des ersten Photometers eingeschaltet wird.

14. Vorrichtung nach einem der Patentansprüche 10 bis 13, dadurch gekennzeichnet, daß die Einimpfvorrichtung aus einer Vorrichtung zum automatischen Dosieren (5) mit einer Betriebskontrolle und einem Ventil (V1) zum Einfüllen des Wassers in einen Behälter (50) besteht, der an seiner Oberseite ein Überlaufrohr (51) und auf seiner Unterseite ein Abfüllrohr (55) mit einem zweiten Ventil (V2) und einer Sammelvorrichtung (2) besteht, die in einen Abfluß (53) mündet.

15. Anwendung des Verfahrens und/oder der Vorrichtung nach einem der obigen Patentansprüche, dadurch gekennzeichnet, daß sie zur Bestimmung der Anwesenheit von Sulfato-Reduktionsbakterien in anaerobem, einem Erdölvorkommen entnommenem Wasser sowie zum anschließenden Auslösen der bakterienbekämpfenden Wirkung dient.
